# EUROPEAN PATENT APPLICATION

(11) **EP 2 599 482 A1**
(43) Date of publication of application: **05.06.2013**
(21) Application number: 11009476.0
(22) Date of filing: 30.11.2011
(51) Int. Cl.: A61K 31/192, A61K 31/215, A61P 25/28, A61P 25/24, A61P 25/00, A61P 3/00, A61P 9/00

(54) **Low dose therapeutic use of glyceryl tri-(4-phenylbutyrate)**

(71) Applicant: Lunamed AG, 7000 Chur (CH)
(72) Inventor: Truog, Peter, 7000 Chur (CH); Buschmann, Helmut H., 52076 Aachen (DE)
(74) Representative: Peters, Hajo

(57) **Abstract**

The present invention relates to the therapeutic use of Glyceryl tri-(4-phenylbutyrate) in the treatment of various diseases and disorders, such as Alzheimer's disease, cancer, depression, or cystic fibrosis. Particularly, the present invention provides Glyceryl tri-(4-phenylbutyrate) at low daily dosis for use in the treatment of various diseases, pharmaceutical compositions and kits comprising Glyceryl tri-(4-phenylbutyrate) at low dosis for the treatment of various diseases, and methods for treatment of Alzheimer's disease involving the administration of Glyceryl tri-(4-phenylbutyrate) at low daily dosis.

## Description

### Field of the invention

The present invention relates to the therapeutic use of Glyceryl tri-(4-phenylbutyrate) in the treatment of various diseases and disorders, such as Alzheimer's disease, cancer, depression, or cystic fibrosis. Particularly, the present invention provides Glyceryl tri-(4-phenylbutyrate) at low daily dosis for use in the treatment of various diseases, pharmaceutical compositions and kits comprising Glyceryl tri-(4-phenylbutyrate) at low dosis for the treatment of various diseases, and methods for treatment of Alzheimer's disease involving the administration of Glyceryl tri-(4-phenylbutyrate) at low daily dosis.

### Background of the invention

4-phenylbutyric acid (4-PB) and its use for the treatment of various clinical conditions and diseases such as benign prostate hyperplasy, cancer, cystic fibrosis, HIV, kidney and liver failure, thalasemia and urea cycle disorders are known.

For example, WO 85/04805 /Brusilow) discloses a process for wast nitrogen removal in human beings, where 4-phenylbutyrate is administered. DE 19,810,383 (Manhart et al.) describes 4-phenylbutyrate as apoptosis inducing agent for neoplastic therapy. WO 93/07866, WO 9510271 or EP725635 (Samid) disclose compositions and methods using phenylacetic acid derivatives for therapy and prevention of a number of pathologies, including cancer, AIDS, anemia, and several beta-chain hemoglobinopathies. WO 9856370 (US 6,207,195 to Walsh et al.) describes therapeutic sodium 4-phenylbutyrate containing nanosperes for the treatment of cystic fibrosis by CFTR gene therapy.

In form of its sodium salt, sodium 4-phenylbutyrate, it is marketed as a drug in the USA and the European Union. Sodium phenylbutyrate is an orphan drug, marketed by Ucyclyd Pharma (Hunt Valley, USA) under the trade name Buphenyl and by Swedish Orphan International (Sweden) as Ammonaps for the treatment of urea cycle disorders.

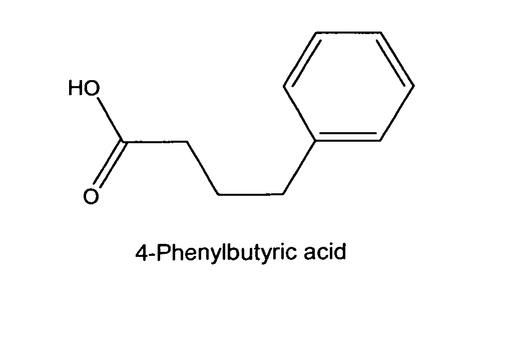

4-phenylbutyric acid is rather quickly broken down in the human body by beta-oxidation to phenylacetic acid. In order to counteract the fast elimination of 4-phenylbutyrate from the body, about 10 to 40 g per patient and day are administered in order to maintain satisfactory blood levels.

However, the currently provided high dosage forms have been shown not to be very effective in the treatment of various diseases and disorders. Therefore, there is a need for improved treatment forms for such diseases and disorders. Moreover, 4-phenylbutyrate is very expensive and has to be taken for several months up to years. Thus, it would be desirable to have a 4-phenylbutyrate formulation that reduces the amount of 4-phenylbutyric acid, while providing satisfactory and effective blood levels.

### Summary of the invention

The present inventors have surprisingly found that Glyceryl tri-(4-phenylbutyrate) (GT4PB) when administered at low dosis ameliorates and effectively treats various diseases and disorders. Moreover, due to its function as depot for 4-phenylbutyric acid which is released after hydrolysis, Glyceryl tri-(4-phenylbutyrate) provides for a suitable slow release profile of 4-PB such that less amounts of 4-PB need to be administered while obtaining satisfactory and effective blood levels thereof.

Based on the above, the present inventors provide the following aspects which summarize the present invention.

One aspect of the invention relates to a pharmaceutical composition comprising Glyceryl tri-(4-phenylbutyrate) in an amount of at most about 750 mg.

Another aspect of the invention relates to Glyceryl tri-(4-phenylbutyrate) for use in the treatment of a disease or disorder, wherein Glyceryl tri-(4-phenylbutyrate) is administered to a subject in need thereof in a daily amount of at most about 750 mg.

Another aspect of the invention relates to Glyceryl tri-(4-phenylbutyrate) for use in the prevention of myocardial infarction or cerebro-vascular diseases, wherein Glyceryl tri-(4-phenylbutyrate) is administered to a subject in need thereof in a daily amount of at most about 750 mg.

Another aspect of the invention relates to a kit for use in the treatment of a disease or disorder, comprising at least one container which contains a pharmaceutical formulation comprising Glyceryl tri-(4-phenylbutyrate) in an amount of at most about 750 mg, and, optionally, at least a second container which contains a pharmaceutical formulation comprising one or more amino acids, such as, e.g. D-amino acid(s).

Another aspect of the invention relates to the use of Glyceryl tri-(4-phenylbutyrate) in the manufacture of a medicament for the treatment of a disease or disorder, wherein Glyceryl tri-(4-phenylbutyrate) is administered to a subject in need thereof in a daily amount of at most about 750 mg.

Another aspect of the invention relates to methods for the treatment of diseases and disorders, comprising administering to a subject in need thereof Glyceryl tri-(4-phenylbutyrate) in a daily amount of at most about 750 mg.

Having summarized the various aspects of the present invention, the invention is now described in more detail. It is intended that each and every embodiment described hereafter may form part of any one of the above aspects. Moreover, it is explicitly intended that the teaching of this invention covers any combination of these embodiments.

### Detailed description of the invention

The present invention provides medicaments, pharmaceutical compositions and pharmaceutical formulations comprising Glyceryl tri-(4-phenylbutyrate) in an amount of at most about 750 mg.

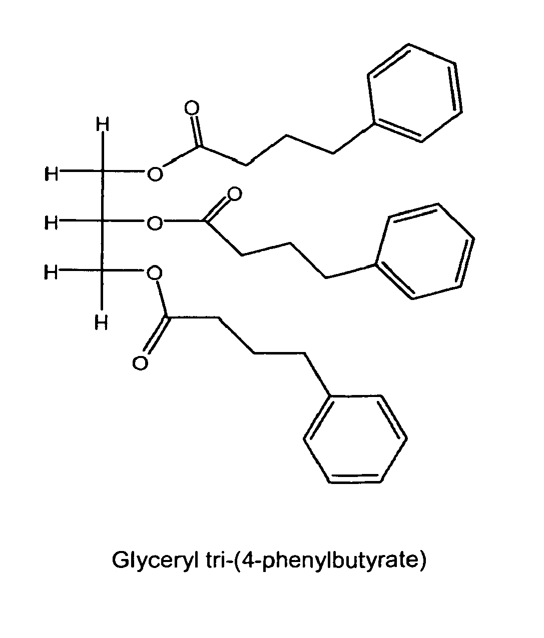

According to the invention, the medicaments, pharmaceutical compositions and pharmaceutical formulations comprise Glyceryl tri-(4-phenylbutyrate) in an amount of at most about 750 mg. The terms "medicament", "pharmaceutical composition" and "pharmaceutical formulation" may be used interchangeably. Suitably, the Glyceryl tri-(4-phenylbutyrate) is present in the medicaments, pharmaceutical compositions and pharmaceutical formulations together with one or more pharmaceutically acceptable carriers, diluents and/or excipients.

Glyceryl tri-(4-phenylbutyrate) can be prepared by standard esterification procedures. It acts as a depot from which 4-PB is slowly release upon metabolization, for instance, in the human digestive system by pancreatic lipase which is able to hydrolyse the triglyceride to produce glycerol and 4-phenylbutyric acid.

Due to the slow release property of Glyceryl tri-(4-phenylbutyrate), the medicament or pharmaceutical composition according to the invention may thus be regarded a slow release medicament or composition. Accordingly, therapeutic blood levels of 4-phenylbutyric acid can be provided over an extended time period. Moreover, fluctuations in 4-phenylbutyric acid blood levels can be reduced. Lastly, the costs of conventional 4-phenylbutyric acid therapy can be reduced.

The medicament or pharmaceutical composition according to the invention can be of any form suitable for the application to humans and/or animals, preferably humans including infants, children and adults and can be produced by standard procedures known to those skilled in the art. The medicament or pharmaceutical composition can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

Carriers, diluents and excipients which are suitable for the preparation of a medicament or pharmaceutical composition according to the present invention are well known to those skilled in the art, e.g. from the "Handbook of Pharmaceutical Excipients" Sixth Edition, Raymond C. Rowe, Paul J. Sheskey and Marian E Quinn (Eds.), American Pharmaceutical Association (July 2009), which is hereby incorporated by reference and forms part of the disclosure.

The medicament or pharmaceutical composition of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments or pharmaceutical formulation may for example be injected intramuscularly, intraperitoneally, or intravenously.

Medicaments or pharmaceutical composition according to the present invention may also be formulated into orally administrable compositions containing one or more pharmaceutically acceptable carriers, diluents and/or excipients. These compositions may contain conventional ingredients such as binding agents, fillers, lubricants, and acceptable wetting agents. The compositions may suitably take the form of capsules, such as, e.g., hard or soft gelatin capsules, lozenges, aqueous or oily solutions, suspensions or emulsions. Medicaments or pharmaceutical composition according to the present invention may also comprise an enteric coating, so that their dissolution is dependent on pH-value. Due to said coating the medicament may pass the stomach undissolved and the active agent is liberated in the intestinal tract. Preferably the enteric coating is soluble at a pH value of 5 to 7.5. Suitable materials and methods for the preparation are known from the prior art.

According to some embodiments, the pharmaceutical composition according to the invention is in form of a sustained release formulation, preferably is in form of an oral sustained release formulation.

By sustained release of the active ingredient is to be understood especially a rate of release of the active ingredient during a period of about 6 to 12 or up to 24 hours.

According to some embodiments, the amount of Glyceryl tri-(4-phenylbutyrate) in the medicament or pharmaceutical composition is at most about 700 mg, at most about 650 mg, at most about 600 mg, at most about 550 mg, at most about 500 mg, at most about 450 mg, at most about 400 mg, at most about 350 mg, at most about 300 mg, at most about 250 mg, at most about 200 mg, at most about 150 mg, at most about 100 mg, at most about 75 mg, at most about 50 mg or at most about 25 mg.

According to some embodiments, the amount of Glyceryl tri-(4-phenylbutyrate) in the medicament or pharmaceutical composition is at most about 100 mg.

According to some embodiments, the amount of Glyceryl tri-(4-phenylbutyrate) in the medicament or pharmaceutical composition is at most about 250 mg.

According to some embodiments, the amount of Glyceryl tri-(4-phenylbutyrate) in the medicament or pharmaceutical composition is at most about 500 mg.

According to some embodiments, the amount of Glyceryl tri-(4-phenylbutyrate) in the medicament or pharmaceutical composition is at least about 25 mg, at least about 50 mg, at least about 75 mg, at least about 100 mg, at least about 150 mg, at least about 200 mg, at least about 250 mg, at least about 300 mg, at least about 350, at least about 400 mg, at least about 450 mg, at least about 500 mg, at least about 550 mg, at least about 600 mg, at least about 650 mg or at least about 700 mg.

According to some embodiments, the amount of Glyceryl tri-(4-phenylbutyrate) in the medicament or pharmaceutical composition is at least about 25 mg.

According to some embodiments, the amount of Glyceryl tri-(4-phenylbutyrate) in the medicament or pharmaceutical composition is at least about 50 mg.

According to some embodiments, the amount of Glyceryl tri-(4-phenylbutyrate) in the medicament or pharmaceutical composition is at least about 75 mg.

According to some embodiments, the amount of Glyceryl tri-(4-phenylbutyrate) in the medicament or pharmaceutical composition is at least about 100 mg.

According to some embodiments, the amount of Glyceryl tri-(4-phenylbutyrate) in the medicament or pharmaceutical composition is at least about 250 mg.

According to some embodiments, the amount of Glyceryl tri-(4-phenylbutyrate) in the medicament or pharmaceutical composition is at least about 500 mg.

According to some embodiments, the amount of Glyceryl tri-(4-phenylbutyrate) in the medicament or pharmaceutical composition is in the range from about 25 mg to 750 mg, from about 50 mg to about 750 mg, from about 75 mg to about 750, about 100 mg to about 750 mg, from about 150 mg to about 750 mg, from about 200 mg to about 750 mg, from about 250 mg to about 750 mg, from about 300 mg to about 750 mg, from about 350 mg to about 750 mg, from about 400 mg to about 750 mg, from about 450 mg to about 750 mg, from about 500 mg to about 750 mg, from about 550 mg to about 750 mg, from about 600 mg to about 750 mg, from about 650 to about 750 mg or from about 700 mg to about 750 mg.

According to some embodiments, the amount of Glyceryl tri-(4-phenylbutyrate) in the medicament or pharmaceutical composition is in the range from about 25 mg to about 500 mg, from about 50 mg to about 500 mg, from about 75 mg to about 500 mg, from about 100 to about 500 mg, from about 150 to about 500 mg, from about 200 mg to about 500 mg, from about 250 mg to about 500 mg, from about 300 mg to about 500 mg, from about 350 mg to about 500 mg, from about 400 mg to about 500 mg or from about 450 to about 500 mg.

According to some embodiments, the amount of Glyceryl tri-(4-phenylbutyrate) in the medicament or pharmaceutical composition is in the range from about 25 mg to about 250 mg, from about 50 mg to about 250 mg, from about 75 mg to about 250 mg, from about 100 to about 250 mg, from about 125 mg to about 250 mg, from about 150 to about 250 mg, from about 175 mg to about 250 mg or from about 200 mg to about 250 mg.

According to some embodiments, the amount of Glyceryl tri-(4-phenylbutyrate) in the medicament or pharmaceutical composition is in the range from about 25 mg to about 100 mg, from about 50 mg to about 100 mg, from about 55 mg to about 100 mg, from about 60 mg to about 100 mg, from about 65 mg to about 100 mg, from about 70 mg to about 100 mg, from about 75 mg to about 100 mg, from about 80 mg to about 100 mg, from about 85 mg to about 100 mg, from about 90 mg to about 100 mg or from about 95 to about 100 mg.

According to some embodiments, the medicament or pharmaceutical composition is for use in the treatment of a disease or disorder selected from cancer, depression, cystic fibrosis, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, Alzheimer's disease, myelodysplastic syndrome, fibrotic lung diseases, hepatic encephalopathies, thioredoxin mediated diseases, human papilloma virus (HPV) infections, autoimmune diseases, thalassemia, genetic disorders such as epigenetic disorders.

According to some embodiments, the medicament or pharmaceutical composition is for use in the treatment of a disease or disorder selected from cancer such as, e.g. prostate cancer, cystic fibrosis, mucositis, amyotropic lateral sclerosis (ALS), skin/mucosal itching, degenerative diseases of the eye, neurodegenerative disorders, Huntington's disease, eating disorders, obesity, drug induced weight gain, metabolic syndrome, diabetes, pruritis, alcoholism, promyelocytic leukemia, especially acute promyelocytic leukemia, cardiac injury, especially adriamycin induced cardiac injury, arteriosclerosis, epithelial damage, connective tissue damage, desmosis, Parkinson Disease, Alzheimer's disease, myelodysplastic syndrome, fibrotic lung diseases, hepatic encephalopathies, thioredoxin mediated diseases, ischemia, multiple sclerosis, chronic vaginal yeast or fungal infection, vaginitis, varicosis, especially varicosis of peripheral veins, spinal muscular atrophy (motor neuron disorders), sickle cell disease, Sudden Infant Death Syndrome, human papilloma virus (HPV) infections, autoimmune diseases, sickle cell diseases, thalassemia, genetic disorders such as epigenetic disorders.

According to some embodiments, the medicament or pharmaceutical composition is for use in the prevention of myocardial infarction or cerebro-vascular diseases, especially wherein Glyceryl tri-(4-phenylbutyrate) is administered to a subject in need thereof in a daily amount of at most about 750 mg.

According to some embodiments, the medicament or pharmaceutical composition is for use in the treatment of Alzheimer's disease.

According to some embodiments, the medicament or pharmaceutical composition according to the invention is for use in the treatment of cancer. According to some specific embodiments, the cancer is prostate cancer. According to other specific embodiments, the cancer is breast cancer. According to other specific embodiments, the cancer is colon cancer.

According to some embodiments, the medicament or pharmaceutical composition according to the invention is for use in the treatment of depression.

According to some embodiments, the medicament or pharmaceutical composition according to the invention is for use in the treatment of leukaemia.

According to some embodiments, the medicament or pharmaceutical composition according to the invention is for use in the treatment of cystic fibrosis.

According to some embodiments, the medicament or pharmaceutical composition according to the invention is for use in the treatment of genetic disorders.

According to some embodiments, the medicament or pharmaceutical composition according to the invention is for use in the treatment of epigenetic disorders.

According to some embodiments, the medicament or pharmaceutical composition further comprises one or more amino acids (such as, e.g. two, three, four, five, six, seven, eight, nine or ten amino acids). The one or more amino acids may be proteinogenic and/or non-proteinogenic. Proteinogenic amino acids, as referred to herein, are those amino acids that can be found in proteins and require cellular machinery coded for in the genetic code of any organism for their isolated production. Proteinogenic amino acids thus include the 22 standard amino acids. Non-proteinogenic amino acids, as referred to herein, are those amino acids which are either not found in proteins, or are not produced directly and in isolation by standard cellular machinery.

According to some embodiments, the one or more amino acids is/are L-amino acid(s) or D-amino acid(s), or a combination thereof.

According to specific embodiments, the one or more amino acids is/ are in the D-configuration.

According to some embodiments, the one or more amino acids is/are selected from nonpolar, polar, acidic or basic amino acids.

According to some embodiments, the one or more amino acids is/are selected from aliphatic, sulfur-containing, aromatic or neutral amino acids.

According some embodiments, the one or more amino acids is/are selected from alanine, valine, leucine, isoleucine, proline, methionine, phenylalanine and tryptophane.

According some embodiments, the one or more amino acids is/are selected from serine, threonine, asparagine, glutamine, cysteine and tyrosine.

According some embodiments, the one or more amino acids is/are selected from aspartic acid and glutamic acid.

According some embodiments, the one or more amino acids is/are selected from lysine, arginine and histidine.

According some embodiments, the one or more amino acids is/are selected from alanine, valine, Leucine and isoleucine.

According some embodiments, the one or more amino acids is/are selected from cysteine and methionine.

According some embodiments, the one or more amino acid is/are selected from phenylalanine, tyrosine and tryptophane.

According some embodiments, the one or more amino acids is/are selected from serine, threonine, asparagine and glutamic acid.

According some embodiments, the one or more amino acids is/are selected from selenocysteine, selenomethionine, norleucine, norvaline, ornithine, penicillamine, hydroxyproline, homoserine, homophenylalanine, homoleucine, homonorleucine and homoarginine,

According some embodiments, the one or more amino acids is selected from alanine, arginine, aspargine, aspartic acid, cysteine, glutamic acid, glutamine, histidine, Isoleucine, leucine, lysine, methionine, phenylalanine, proline, serine, threonine, tryptophane, tyrosine, valine, selenocysteine, selenomethionine, norleucine, norvaline, ornithine, penicillamine, hydroxyproline, homoserine, homophenylalanine, homonorleucine and homoarginine.

According some embodiments, the one amino acid is alanine, especially D-alanine.

According some embodiments, the one or more amino acids is/are not carnitine, homocystein and canavanine, neither in the L- nor in the D-configuration.

According to some embodiments, the amount of the one or more amino acids in the medicament or pharmaceutical composition is in the range from about 1:10 to about 10:1 w/w of the amount of Glyceryl tri-(4-phenylbutyrate), each or total.

According to some embodiments, the amount of the one or more amino acids in the medicament or pharmaceutical composition is in the range from about 1:5 to about 5:1 w/w of the amount of Glyceryl tri-(4-phenylbutyrate), each or total.

According to some embodiments, the amount of the one or more amino acids in the medicament or pharmaceutical composition is in the range from about 1:2 to about 2:1 w/w of the amount of Glyceryl tri-(4-phenylbutyrate), each or total.

According to some embodiments, the amount of the one or more amino acids in the medicament or pharmaceutical composition is 1:1 w/w of the amount of Glyceryl tri-(4-phenylbutyrate), each or total.

According to some embodiments, the medicament or pharmaceutical composition according to the invention is administered one or more times a day, such as, e.g., two times, three times, four times or five times. According to some embodiments, the medicament or pharmaceutical composition according to the invention is administered once a day. According to some embodiments, the medicament or pharmaceutical composition according to the invention is administered twice a day. According to some embodiments, the medicament or pharmaceutical composition according to the invention is administered once in the morning and once in the evening.

According to some embodiments, the medicament or pharmaceutical composition according to the invention is administered two times or more a day, such as, e.g., three times, four times or five times, wherein the administrations are 2 to 14 hours apart, such as, e.g., 4, 6, 8, 10 or 12 hours apart. According to specific embodiments, the medicament or pharmaceutical composition according to the invention is administered twice a day, wherein the administrations are 8 to 12 hours apart.

The present invention further provides Glyceryl tri-(4-phenylbutyrate) for use in the treatment of a disease or disorder. According to the invention, Glyceryl tri-(4-phenylbutyrate) is administered to a subject in need thereof in a daily amount of at most about 750 mg.

According to the invention, a subject or patient is an organism that exhibits the properties or symptoms associated with Alzheimer's disease. The subject or patient is typically a vertebrate, in particular a mammal. According to some embodiments, the subject or patient is a human (Homo sapiens). The terms "subject" and "patient" may be used interchangeably.

According to some embodiments, the disease or disorder is selected from cancer, depression, cystic fibrosis, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, Alzheimer's disease, myelodysplastic syndrome, fibrotic lung diseases, hepatic encephalopathies, thioredoxin mediated diseases, human papilloma virus (HPV) infections, autoimmune diseases, thalassemia, genetic disorders such as epigenetic disorders.

According to some embodiments, the Glyceryl tri-(4-phenylbutyrate) is for use in the treatment of Alzheimer's disease.

According to some embodiments, the Glyceryl tri-(4-phenylbutyrate) is for use in the treatment of cancer. According to some specific embodiments, the cancer is prostate cancer. According to other specific embodiments, the cancer is breast cancer. According to other specific embodiments, the cancer is colon cancer.

According to some embodiments, the Glyceryl tri-(4-phenylbutyrate) is for use in the treatment of depression.

According to some embodiments, the Glyceryl tri-(4-phenylbutyrate) is for use in the treatment of leukaemia.

According to some embodiments, the Glyceryl tri-(4-phenylbutyrate) is for use in the treatment of cystic fibrosis.

According to some embodiments, the Glyceryl tri-(4-phenylbutyrate) is for use in the treatment of genetic disorders.

According to some embodiments, the Glyceryl tri-(4-phenylbutyrate) is for use in the treatment of epigenetic disorders.

According to some embodiments, the daily amount of Glyceryl tri-(4-phenylbutyrate) to be administered may be at most about 700 mg, at most about 650 mg, at most about 600 mg, at most about 550 mg, at most about 500 mg, at most about 450 mg, at most about 400 mg, at most about 350 mg, at most about 300 mg, at most about 250 mg, at most about 200 mg, at most about 150 mg, at most about 100 mg, at most about 75 mg, at most about 50 mg or at most about 25 mg.

According to some embodiments, the daily amount of Glyceryl tri-(4-phenylbutyrate) to be administered is at most about 100 mg

According to some embodiments, the daily amount of Glyceryl tri-(4-phenylbutyrate) to be administered is at most about 250 mg.

According to some embodiments, the daily amount of Glyceryl tri-(4-phenylbutyrate) to be administered is at most about 500 mg.

According to some embodiments, the daily amount of Glyceryl tri-(4-phenylbutyrate) to be administered is at least about 25 mg, at least about 50 mg, at least about 75 mg, at least about 100 mg, at least about 150 mg, at least about 200 mg, at least about 250 mg, at least about 300 mg, at least about 350, at least about 400 mg, at least about 450 mg, at least about 500 mg, at least about 550 mg, at least about 600 mg, at least about 650 mg or at least about 700 mg.

According to some embodiments, the daily amount of Glyceryl tri-(4-phenylbutyrate) to be administered is at least about 25 mg.

According to some embodiments, the daily amount of Glyceryl tri-(4-phenylbutyrate) to be administered is at least about 50 mg.

According to some embodiments, the daily amount of Glyceryl tri-(4-phenylbutyrate) to be administered is at least about 75 mg.

According to some embodiments, the daily amount of Glyceryl tri-(4-phenylbutyrate) to be administered is at least about 100 mg.

According to some embodiments, the daily amount of Glyceryl tri-(4-phenylbutyrate) to be administered is at least about 250 mg.

According to some embodiments, the daily amount of Glyceryl tri-(4-phenylbutyrate) to be administered is at least about 500 mg.

According to some embodiments, the daily amount of Glyceryl tri-(4-phenylbutyrate) to be administered is in the range from about 25 mg to about 750 mg, from about 50 mg to about 750 mg, from about 75 mg to about 750, about 100 mg to about 750 mg, from about 150 mg to about 750 mg, from about 200 mg to about 750 mg, from about 250 mg to about 750 mg, from about 300 mg to about 750 mg, from about 350 mg to about 750 mg, from about 400 mg to about 750 mg, from about 450 mg to about 750 mg, from about 500 mg to about 750 mg, from about 550 mg to about 750 mg, from about 600 mg to about 750 mg, from about 650 to about 750 mg or from about 700 mg to about 750 mg.

According to some embodiments, the daily amount of Glyceryl tri-(4-phenylbutyrate) to be administered is in the range from about 25 mg to about 500 mg, from about 50 mg to about 500 mg, from about 75 mg to about 500 mg, from about 100 to about 500 mg, from about 150 to about 500 mg, from about 200 mg to about 500 mg, from about 250 mg to about 500 mg, from about 300 mg to about 500 mg, from about 350 mg to about 500 mg, from about 400 mg to about 500 mg or from about 450 to about 500 mg.

According to some embodiments, the daily amount of Glyceryl tri-(4-phenylbutyrate) to be administered is in the range from about 25 mg to about 250 mg, from about 50 mg to about 250 mg, from about 75 mg to about 250 mg, from about 100 to about 250 mg, from about 125 mg to about 250 mg, from about 150 to about 250 mg, from about 175 mg to about 250 mg or from about 200 mg to about 250 mg.

According to some embodiments, the daily amount of Glyceryl tri-(4-phenylbutyrate) to be administered is in the range from about 25 mg to about 100 mg, from about 50 mg to about 100 mg, from about 55 mg to about 100 mg, from about 60 mg to about 100 mg, from about 65 mg to about 100 mg, from about 70 mg to about 100 mg, from about 75 mg to about 100 mg, from about 80 mg to about 100 mg, from about 85 mg to about 100 mg, from about 90 mg to about 100 mg or from about 95 to about 100 mg.

According to some embodiments, the Glyceryl tri-(4-phenylbutyrate) is administered one or more times a day, such as, e.g., two times, three times, four times or five times. According to some embodiments, the Glyceryl tri-(4-phenylbutyrate) is administered once a day. According to some embodiments, the Glyceryl tri-(4-phenylbutyrate) is administered twice a day. According to some embodiments, the Glyceryl tri-(4-phenylbutyrate) is administered once in the morning and once in the evening.

According to some embodiments, Glyceryl tri-(4-phenylbutyrate) is administered two times or more a day, such as, e.g., three times, four times or five times, wherein the administrations are 2 to 14 hours apart, such as, e.g., 4, 6, 8, 10 or 12 hours apart. According to specific embodiments, the Glyceryl tri-(4-phenylbutyrate) is administered twice a day, wherein the administrations are 8 to 12 hours apart.

According to some embodiments, Glyceryl tri-(4-phenylbutyrate) is administered in combination with one or more amino acids (such as, e.g. two, three, four, five, six, seven, eight, nine or ten amino acids).

According to some embodiments, the one or more amino acids are L-amino acids or D-amino acids, or a combination thereof.

According to specific embodiments, the one or more amino acids is/are in the D-configuration.

According to some embodiments, the one or more amino acids is/are selected from nonpolar, polar, acidic or basic amino acids.

According to some embodiments, the one or more amino acids is/are selected from aliphatic, sulfur-containing, aromatic or neutral amino acids.

According to some embodiments, the one or more amino acids is/are selected from alanine, valine, leucine, isoleucine, proline, methionine, phenylalanine and tryptophane.

According to some embodiments, the one or more amino acids is/are selected from serine, threonine, asparagine, glutamine, cysteine and tyrosine.

According to some embodiments, the one or more amino acids is/are selected from aspartic acid and glutamic acid.

According to some embodiments, the one or more amino acids is/are selected from lysine, arginine and histidine.

According to some embodiments, the one or more amino acids is/are selected from alanine, valine, Leucine and isoleucine.

According to some embodiments, the one or more amino acids is/are selected from cysteine and methionine.

According to some embodiments, the one or more amino acid is/are selected from phenylalanine, tyrosine and tryptophane.

According to some embodiments, the one or more amino acid is/are selected from serine, threonine, asparagine and glutamic acid.

According to some embodiments, the one or more amino acid is/are selected from selenocysteine, selenomethionine, norleucine, norvaline, ornithine, penicillamine, hydroxyproline, homoserine, homophenylalanine, homoleucine, homonorleucine and homoarginine,

According to some embodiments, the one or more amino acid is selected from alanine, arginine, aspargine, aspartic acid, cysteine, glutamic acid, glutamine, histidine, Isoleucine, leucine, lysine, methionine, phenylalanine, proline, serine, threonine, tryptophane, tyrosine, valine, selenocysteine, selenomethionine, norleucine, norvaline, ornithine, penicillamine, hydroxyproline, homoserine, homophenylalanine, homonorleucine and homoarginine.

According to some embodiments, the one amino acid is alanine, especially D-alanine.

According to some embodiments, the one or more amino acids is/are not carnitine, homocystein and canavanine, neither in the L- nor in the D-configuration.

According to some embodiments, the amount of the one or more amino acids to be administered in combination with Glyceryl tri-(4-phenylbutyrate) is in the range from about 1:10 to about 10:1 w/w of the amount of Glyceryl tri-(4-phenylbutyrate), each or total.

According to some embodiments, the amount of the one or more amino acids to be administered in combination with Glyceryl tri-(4-phenylbutyrate) is in the range from about 1:5 to about 5:1 w/w of the amount of Glyceryl tri-(4-phenylbutyrate), each or total.

According to some embodiments, the amount of the one or more amino acids to be administered in combination with Glyceryl tri-(4-phenylbutyrate) is in the range from about 1:2 to about 2:1 w/w of the amount of Glyceryl tri-(4-phenylbutyrate), each or total.

According to some embodiments, the amount of the one or more amino acids to be administered in combination with Glyceryl tri-(4-phenylbutyrate) is 1:1 w/w of the amount of Glyceryl tri-(4-phenylbutyrate), each or total.

The present invention further provides kits for use in the treatment of a disease or disorder. According to the invention, the kits comprise at least one (first) container which contains a pharmaceutical formulation comprising Glyceryl tri-(4-phenylbutyrate) in an amount of at most about 750 mg. Suitably, Glyceryl tri-(4-phenylbutyrate) is present in the formulation together with one or more pharmaceutically acceptable carriers, diluents and/or excipients. For further details on pharmaceutically acceptable carriers, diluents and excipients reference is made to above description.

According to some embodiments, the disease or disorder is selected from cancer, depression, cystic fibrosis, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, Alzheimer's disease, myelodysplastic syndrome, fibrotic lung diseases, hepatic encephalopathies, thioredoxin mediated diseases, human papilloma virus (HPV) infections, autoimmune diseases, thalassemia, genetic disorders such as epigenetic disorders.

According to some embodiments, the kit is for use in the treatment of Alzheimer's disease.

According to some embodiments, the kit is for use in the treatment of cancer. According to some specific embodiments, the cancer is prostate cancer. According to other specific embodiments, the cancer is breast cancer. According to other specific embodiments, the cancer is colon cancer.

According to some embodiments, the kit is for use in the treatment of depression.

According to some embodiments, the kit is for use in the treatment of leukaemia.

According to some embodiments, the kit is for use in the treatment of cystic fibrosis.

According to some embodiments, the kit is for use in the treatment of genetic disorders.

According to some embodiments, the kit is for use in the treatment of epigenetic disorders.

According to some embodiments, the amount of Glyceryl tri-(4-phenylbutyrate) comprised by the pharmaceutical formulation is at most about 700 mg, at most about 650 mg, at most about 600 mg, at most about 550 mg, at most about 500 mg, at most about 450 mg, at most about 400 mg, at most about 350 mg, at most about 300 mg, at most about 250 mg, at most about 200 mg, at most about 150 mg, at most about 100 mg, at most about 75 mg, at most about 50 mg or at most about 25 mg.

According to some embodiments, the amount of Glyceryl tri-(4-phenylbutyrate) comprised by the pharmaceutical formulation is at most about 100 mg

According to some embodiments, the amount of Glyceryl tri-(4-phenylbutyrate) comprised by the pharmaceutical formulation is at most about 250 mg.

According to some embodiments, the amount of Glyceryl tri-(4-phenylbutyrate) comprised by the pharmaceutical formulation is at most about 500 mg.

According to some embodiments, the amount of Glyceryl tri-(4-phenylbutyrate) comprised by the pharmaceutical formulation is at least about 25 mg, at least about 50 mg, at least about 75 mg, at least about 100 mg, at least about 150 mg, at least about 200 mg, at least about 250 mg, at least about 300 mg, at least about 350, at least about 400 mg, at least about 450 mg, at least about 500 mg, at least about 550 mg, at least about 600 mg, at least about 650 mg or at least about 700 mg.

According to some embodiments, the amount of Glyceryl tri-(4-phenylbutyrate) comprised by the pharmaceutical formulation is at least about 25 mg.

According to some embodiments, the amount of Glyceryl tri-(4-phenylbutyrate) comprised by the pharmaceutical formulation is at least about 50 mg.

According to some embodiments, the amount of Glyceryl tri-(4-phenylbutyrate) comprised by the pharmaceutical formulation is at least about 75 mg.

According to some embodiments, the amount of Glyceryl tri-(4-phenylbutyrate) comprised by the pharmaceutical formulation is at least about 100 mg.

According to some embodiments, the amount of Glyceryl tri-(4-phenylbutyrate) comprised by the pharmaceutical formulation is at least about 250 mg.

According to some embodiments, the amount of Glyceryl tri-(4-phenylbutyrate) comprised by the pharmaceutical formulation is at least about 500 mg.

According to some embodiments, the amount of Glyceryl tri-(4-phenylbutyrate) comprised by the pharmaceutical formulation is in the range from about 25 mg to about 750 mg, from about 50 mg to about 750 mg, from about 75 mg to about 750, about 100 mg to about 750 mg, from about 150 mg to about 750 mg, from about 200 mg to about 750 mg, from about 250 mg to about 750 mg, from about 300 mg to about 750 mg, from about 350 mg to about 750 mg, from about 400 mg to about 750 mg, from about 450 mg to about 750 mg, from about 500 mg to about 750 mg, from about 550 mg to about 750 mg, from about 600 mg to about 750 mg, from about 650 to about 750 mg or from about 700 mg to about 750 mg.

According to some embodiments, the amount of Glyceryl tri-(4-phenylbutyrate) comprised by the pharmaceutical formulation is in the range from about 25 mg to about 500 mg, from about 50 mg to about 500 mg, from about 75 mg to about 500 mg, from about 100 to about 500 mg, from about 150 to about 500 mg, from about 200 mg to about 500 mg, from about 250 mg to about 500 mg, from about 300 mg to about 500 mg, from about 350 mg to about 500 mg, from about 400 mg to about 500 mg or from about 450 to about 500 mg.

According to some embodiments, the amount of Glyceryl tri-(4-phenylbutyrate) comprised by the pharmaceutical formulation is in the range from about 25 mg to about 250 mg, from about 50 mg to about 250 mg, from about 75 mg to about 250 mg, from about 100 to about 250 mg, from about 125 mg to about 250 mg, from about 150 to about 250 mg, from about 175 mg to about 250 mg or from about 200 mg to about 250 mg.

According to some embodiments, the amount of Glyceryl tri-(4-phenylbutyrate) comprised by the pharmaceutical formulation is in the range from about 25 mg to about 100 mg, from about 50 mg to about 100 mg, from about 55 mg to about 100 mg, from about 60 mg to about 100 mg, from about 65 mg to about 100 mg, from about 70 mg to about 100 mg, from about 75 mg to about 100 mg, from about 80 mg to about 100 mg, from about 85 mg to about 100 mg, from about 90 mg to about 100 mg or from about 95 to about 100 mg.

According to some embodiments, the kits further comprise at least a further (such as, e.g., a second, third, fourth, etc.) container which contains a pharmaceutical formulation comprising one or more amino acids (such as, e.g. two, three, four, five, six, seven, eight, nine or ten amino acids). Suitably, the one or more amino acids is/are present in the pharmaceutical formulation together with one or more pharmaceutically acceptable carriers, diluents and/or excipients. For further details on pharmaceutically acceptable carriers, diluents and excipients reference is made to above description.

According to some embodiments, the one or more amino acids is/are L-amino acid(s) or D-amino acid(s), or a combination thereof.

According to specific embodiments, the one or more amino acids is/ are in the D-configuration.

According to some embodiments, the one or more amino acids is/are selected from nonpolar, polar, acidic or basic amino acids.

According to some embodiments, the one or more amino acids is/are selected from aliphatic, sulfur-containing, aromatic or neutral amino acids.

According some embodiments, the one or more amino acids is/are selected from alanine, valine, leucine, isoleucine, proline, methionine, phenylalanine and tryptophane.

According some embodiments, the one or more amino acids is/are selected from serine, threonine, asparagine, glutamine, cysteine and tyrosine.

According some embodiments, the one or more amino acids is/are selected from aspartic acid and glutamic acid.

According some embodiments, the one or more amino acids is/are selected from lysine, arginine and histidine.

According some embodiments, the one or more amino acids is/are selected from alanine, valine, Leucine and isoleucine.

According some embodiments, the one or more amino acids is/are selected from cysteine and methionine.

According some embodiments, the one or more amino acid is/are selected from phenylalanine, tyrosine and tryptophane.

According some embodiments, the one or more amino acid is/are selected from serine, threonine, asparagine and glutamic acid.

According some embodiments, the one or more amino acid is/are selected from selenocysteine, selenomethionine, norleucine, norvaline, ornithine, penicillamine, hydroxyproline, homoserine, homophenylalanine, homoleucine, homonorleucine and homoarginine,

According some embodiments, the one or more amino acid is selected from alanine, arginine, aspargine, aspartic acid, cysteine, glutamic acid, glutamine, histidine, Isoleucine, leucine, lysine, methionine, phenylalanine, proline, serine, threonine, tryptophane, tyrosine, valine, selenocysteine, selenomethionine, norleucine, norvaline, ornithine, penicillamine, hydroxyproline, homoserine, homophenylalanine, homonorleucine and homoarginine.

According some embodiments, the one amino acid is alanine, especially D-alanine.

According some embodiments, the one or more amino acids is/are not carnitine, homocystein and canavanine, neither in the L- nor in the D-configuration.

According to some embodiments, the amount of the one or more amino acids in the pharmaceutical formulation is in the range from about 1:10 to about 10:1 w/w of the amount of Glyceryl tri-(4-phenylbutyrate), each or total.

According to some embodiments, the amount of the one or more amino acids in the pharmaceutical formulation is in the range from about 1:5 to about 5:1 w/w of the amount of Glyceryl tri-(4-phenylbutyrate), each or total.

According to some embodiments, the amount of the one or more amino acids in the pharmaceutical formulation is in the range from about 1:2 to about 2:1 w/w of the amount of Glyceryl tri-(4-phenylbutyrate), each or total.

According to some embodiments, the amount of the one or more amino acids in the pharmaceutical formulation is 1:1 w/w of the amount of Glyceryl tri-(4-phenylbutyrate), each or total.

Where more than one amino acid is comprised by the kit, each amino acid may be presented within an individual pharmaceutical formulation contained in a separate container.

According to some embodiments, also the medicaments, pharmaceutical compositions and pharmaceutical formulations as disclosed herein in the context of other aspects of the present invention may form part of a kit according to the invention, and as such may be comprised within one or more containers of such kit.

It is again explicitly intended that the teaching of this invention covers any combination of the above described embodiments.

## Claims

1. A pharmaceutical composition comprising Glyceryl tri-(4-phenylbutyrate) in an amount of at most about 750 mg.

2. The pharmaceutical composition according to claim 1, wherein the amount of Glyceryl tri-(4-phenylbutyrate) it at most about 700 mg, at most about 650 mg, at most about 600 mg, at most about 550 mg, at most about 500 mg, at most about 450 mg, at most about 400 mg, at most about 350 mg, at most about 300 mg, at most about 250 mg, at most about 200 mg, at most about 150 mg, at most about 100 mg, at most about 75 mg, at most about 50 mg or at most about 25 mg.

3. The pharmaceutical composition according to claim 1 or 2, wherein the amount of Glyceryl tri-(4-phenylbutyrate) is at most about 250 mg or 500 mg.

4. The pharmaceutical composition according to any one of claims 1 to 3, wherein the amount of 4-PB is at least about 25 mg.

5. The pharmaceutical composition according to any one of claims 1 to 4 for use in the treatment of a disease or disorder selected from cancer such as, e.g. prostate cancer, cystic fibrosis, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, Alzheimer's disease, myelodysplastic syndrome, fibrotic lung diseases, hepatic encephalopathies, thioredoxin mediated diseases, human papilloma virus (HPV) infections, autoimmune diseases, thalassemia, genetic disorders such as epigenetic disorders.

6. The pharmaceutical composition according to any one of claims 1 to 4 for use in the treatment of Alzheimer's disease.

7. The pharmaceutical composition according to any one of claims 1 to 4 for use in the treatment of depression.

8. Glyceryl tri-(4-phenylbutyrate) for use in the treatment of a disease or disorder, wherein Glyceryl tri-(4-phenylbutyrate) is administered to a subject in need thereof in a daily amount of at most about 750 mg.

9. Glyceryl tri-(4-phenylbutyrate) for use in the prevention of myocardial infarction or cerebro-vascular diseases, wherein Glyceryl tri-(4-phenylbutyrate) is administered to a subject in need thereof in a daily amount of at most about 750 mg.

10. Glyceryl tri-(4-phenylbutyrate) for use according to any of claims 8 or 9, wherein the daily amount of Glyceryl tri-(4-phenylbutyrate) to be administered is at most about 700 mg, at most about 650 mg, at most about 600 mg, at most about 550 mg, at most about 500 mg, at most about 450 mg, at most about 400 mg, at most about 350 mg, at most about 300 mg, at most about 250 mg, at most about 200 mg, at most about 150 mg, at most about 100 mg, at most about 75 mg, at most about 50 mg or at most about 25 mg.

11. Glyceryl tri-(4-phenylbutyrate) for use according to any one of claims 8 to 10, wherein the daily amount of Glyceryl tri-(4-phenylbutyrate) to be administered is at most about 250 mg or 500 mg.

12. Glyceryl tri-(4-phenylbutyrate) for use according to any one of claims 8 to 11, wherein the daily amount of Glyceryl tri-(4-phenylbutyrate) is at least about 50 mg or 25 mg.

13. Glyceryl tri-(4-phenylbutyrate) for use according to any one of claims 8, 10 to 12, wherein the disease or disorder is selected from cancer such as, e.g. prostate cancer, cystic fibrosis, mucositis, amyotropic lateral sclerosis (ALS), skin/mucosal itching, degenerative diseases of the eye, neurodegenerative disorders, Huntington's disease, eating disorders, obesity, drug induced weight gain, metabolic syndrome, diabetes, pruritis, alcoholism, promyelocytic leukemia, especially acute promyelocytic leukemia, cardiac injury, especially adriamycin induced cardiac injury, arteriosclerosis, epithelial damage, connective tissue damage, desmosis, Parkinson Disease, Alzheimer's disease, myelodysplastic syndrome, fibrotic lung diseases, hepatic encephalopathies, thioredoxin mediated diseases, ischemia, multiple sclerosis, chronic vaginal yeast or fungal infection, vaginitis, varicosis, especially varicosis of peripheral veins, spinal muscular atrophy (motor neuron disorders), sickle cell disease, Sudden Infant Death Syndrome, human papilloma virus (HPV) infections, autoimmune diseases, sickle cell diseases, thalassemia, genetic disorders such as epigenetic disorders.

14. Glyceryl tri-(4-phenylbutyrate) for use according to any one of claims 8 to 11, wherein the disease or disorder is Alzheimer's disease or wherein the disease or disorder is depression.

15. Kit for use in the treatment of a disease or disorder, comprising at least one container which contains a pharmaceutical formulation comprising Glyceryl tri-(4-phenylbutyrate) in an amount of at most about 750 mg, and, optionally, at least a second container which contains a pharmaceutical formulation comprising one or more amino acids, such as, e.g. D-amino acid(s).
